# EUROPÄISCHE PATENTSCHRIFT

(11) **EP 0 510 490 B1**
(45) Veröffentlichungstag und Bekanntmachung des Hinweises auf die Patenterteilung: **15.02.1995**
(21) Anmeldenummer: 92106418.4
(22) Anmeldetag: 14.04.1992
(51) Int. Cl.: C07C 51/00, C07C 63/70

(54) **Verfahren zur Herstellung von 2,3,4,5-Tetrafluorbenzoesäure**
Process for preparing 2,3,4,5-tetrafluorobenzoic acid
Procédé de préparation d'acide 2,3,4,5-tetrafluorobenzoique

(30) Priorität: 25.04.1991 DE 4113462
(43) Veröffentlichungstag der Anmeldung: 28.10.1992
(73) Patentinhaber: HOECHST AKTIENGESELLSCHAFT, 65926 Frankfurt am Main (DE)
(72) Erfinder: Papenfuhs, Theodor, Dr., W-6000 Frankfurt am Main 50 (DE); Pfirmann, Ralf, Dr., W-6103 Griesheim (DE)

(56) Entgegenhaltungen:
- EP-A- 0 194 671
- EP-A- 0 259 663
- CHEMICAL ABSTRACTS, vol. 108, no. 1, 4. Januar 1988, Columbus, Ohio, US;abstract no. 5686, Seite 537 ;
- HOUBEN-WEYL 'METHODEN DER ORGANISCHEN CHEMIE, BAND X/2,"STICKSTOFFVERBINDUNGENI.' 1967 , GEORG THIEME VERLAG , STUTTGART,DE

## Beschreibung

Die vorliegende Erfindung betrifft ein verbessertes Verfahren zur Herstellung von 2,3,4,5-Tetrafluorbenzoesäure aus N'-substituierten N-Amino-3,4,5,6-Tetrafluorphthalimiden, die durch saure Hydrolyse unter gleichzeitiger Decarboxylierung in die 2,3,4,5-Tetrafluorbenzoesäure, ein zentrales Zwischenprodukt zur Herstellung antibakterieller Mittel, umgewandelt werden können (DE-A 3318145).

2,3,4,5-Tetrafluorbenzoesäure konnte bisher aus Tetrachlorphthaloylchlorid (G. G. Yakobson, V. N. Odinkov, N. N. Vorozhtsov, Zh. Obshsh. Khim. 36 (1966), 139; Imperial Chemical Industries PLC, EP 140482, GB 2146635, 24.7.84), aus Tetrafluor anthranilsäure (S. Hayashi, N. Ishikawa, Bull. Chem. Soc. Jap. 45 (1972), 2909), aus 1,2,3,4-Tetrafluorbenzol (L. J. Belf, M. W. Buxton, J. F. Tilney-Bassett, Tetrahedron 23 (1967), 4719; Z. Naturforsch. 31B (1976), 1667), aus Tetrachlorphthalsäureanhydrid (Bayer AG, DE 3810093 A1, 5.10.89; Warner-Lambert Co., EP 218111, 9.9.86) oder aus Tetrachlorphthalodinitril (Imperial Chemical Industries PLC, GB 2134900, 22.8.84) über teilweise aufwendige und/oder technisch nicht zu verwirklichende Schritte dargestellt werden. Dieselbe Aussage kann für die Herstellung der 2,3,4,5-Tetrafluorbenzoesäure aus 1,2-Dibromtetrafluorbenzol (C. Tamborski, E. J. Soloski, J. Organometallic Chem. 10 (1967), 385) und die von P. Sartori und A. Golloch (Chem. Ber. 101 (1986), 2004) beschriebene Methode, ausgehend von Tetrafluorphthalsäure, getroffen werden. Ebenfalls eingesetzt wurden N-kohlenstoffsubstituerte Tetrachlorphthalimide zur Synthese von Tetrafluorphthalsäure (SDS Biotech K. K., EP 259663, 18.8.87), die in die 2,3,4,5-Tetrafluorbenzoesäure umgewandelt werden kann.

Aus Tetrafluorphthalsäure bzw. deren Anhydrid kann 2,3,4,5-Tetrafluorbenzoesäure nach verschiedenen Verfahren erhalten werden (EP 194 671; EP 218 111; JP 01/025 737; JP 63/295 529). Diese Verfahren laufen zum Teil unter Verwendung technisch nicht verfügbarer oder ökologisch bedenklicher Reagenzien ab. Das Hauptproblem stellt meist die Notwendigkeit der Isolierung von Tetrafluorphthalsäure dar, was erhebliche Schwierigkeiten bereiten kann, bevor diese weiter umgesetzt werden.

Es bestand somit ein Bedürfnis nach einer besseren Herstellungsmöglichkeit für die 2,3,4,5-Tetrafluorbenzoesäure, das durch das erfindungsgemäße Verfahren befriedigt werden konnte.

Gegenstand der vorliegenden Erfindung ist ein verbessertes Verfahren zur Herstellung von 2,3,4,5-Tetrafluorbenzoesäure der Formel (1) 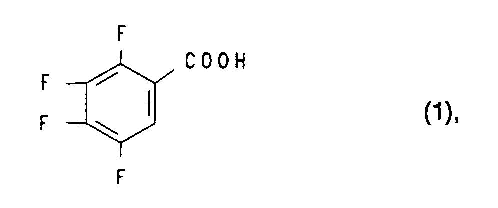
indem man N'-substituierte N-Aminotetrafluorphthalimide der allgemeinen Formel (2) 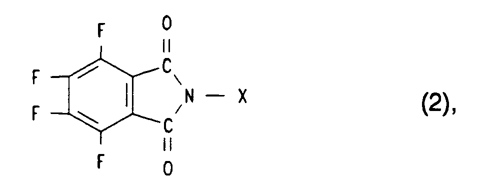
in welcher X den Rest 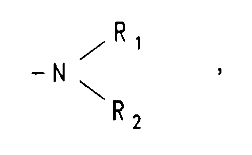
worin R₁ , R₂ je ein Wasserstoffatom, eine Alkyl (C₁-C₁₀)-Gruppe, Arylgruppe, beispielsweise die Phenylgruppe, eine Alkyl (C₁-C₆)-CO-Gruppe, beispielsweise die Acetylgruppe, eine Aryl-CO-Gruppe, beispielsweise die Benzoylgruppe, bedeuten, wobei die Aryl- bzw. Aryl-CO-Gruppen für R₁ und R₂ am aromatischen Kern beispielsweise durch Fluor- und/oder Chloratome und/oder Alkyl-(C₁-C₄)-Gruppen substituiert sein können, oder R₁ und R₂ zusammen einen Phthaloylrest, der am aromatischen Ring durch 4 Chloratome oder 4 Fluoratome substituiert sein kann, vorzugsweise den Rest 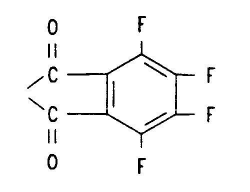
bedeuten,
oder in welcher X den Rest 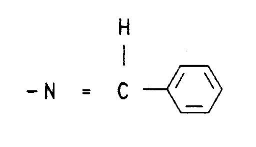
darstellt,
welcher am aromatischen Kern beispielsweise durch Fluor- und/oder Chloratome und/oder Alkyl-(C₁-C₄)-Gruppen substituiert sein kann, in wäßrigem Medium bei pH-Werten von -1 bis +1, vorzugsweise von -0,2 bis +0,4, bei Temperaturen von 160 bis 220 °C, vorzugsweise von 190 bis 205 °C, in einem geschlossenen Behälter (Reaktor) erhitzt.

Die Einstellung des gewünschten sauren pH-Werts der Reaktionsmischung kann sowohl mit organischen Säuren, wie beispielsweise Trifluoressigsäure, Alkyl- oder Arylsulfonsäuren, wie beispielsweise Methansulfonsäure, Trifluormethansulfonsäure, Hexafluorpropansulfonsäure oder p-Toluolsulfonsäure, als auch anorganischen Mineralsäuren, wie beispielsweise Salzsäure, Bromwasserstoffsäure, Jodwasserstoffsäure, Schwefelsäure, Chlorsulfonsäure oder Phosphorsäure vorgenommen werden.

Das erfindungsgemäße Einstufenverfahren hat den Vorteil, daß eine Zwischenisolierung des Tetrafluorphthalsäureanhydrides bzw. der Tetrafluorphthalsäure, was je nach Verfahren zum Teil erhebliche Probleme bereitet, entfällt. Die 2,3,4,5-Tetrafluorbenzoesäure ist hingegen in wäßrigen Medien nur wenig löslich und kann durch Filtration als Rohprodukt erhalten werden.

Da bei dieser Reaktion Kohlendioxid als Reaktionsgas entsteht, hängt der bei der Verfahrensdurchführung auftretende Druck vom Füllstand des Behälters (Reaktor), der Löslichkeit des Kohlendioxids im Reaktionsmedium bei der jeweiligen Reaktionstemperatur und vom Realverhalten des Gases ab. Es wird jedoch stets mindestens der zur Reaktionstemperatur gehörige Wasserdampfdruck erreicht, d.h. ein Druck zwischen etwa 6,1 bar (160 °C) und etwa 22,9 bar (220 °C).

Zur Verhinderung von Korrosion an den verwendeten Reaktionsapparaten durch während der Reaktion entstehendes freies Fluorid, das bei den niedrigen pH-Werten als Fluorwasserstoff vorliegt, kann unter Anwesenheit von fluoridfangenden Mitteln gearbeitet werden. Als solche können Calciumsalze und Siliciumdioxid, vorzugsweise Siliciumdioxid mit hoher innerer Oberfläche, verwendet werden.
Möglich ist ebenfalls die Verwendung von Trialkyl (C₂-C₆)-zinnchloriden, die mit Fluoriden polymere Trialkylzinnfluoride ergeben. Bevorzugt verwendet werden Calciumsalze, wie Calciumhydroxid, das unter den Reaktionsbedingungen als Salz der verwendeten Säure vorliegt, Calciumsulfat, Calciumcarbonat und Calciumchlorid, sowie Siliciumdioxid hoher innerer Oberfläche (®Aerosil).

Eine Reinigung der erfindungsgemäß erhaltenen 2,3,4,5-Tetrafluorbenzoesäure, die gemäß der weiter oben zitierten Literatur bisher stets durch Kristallisation aus verschiedenen Lösungsmitteln erfolgte, kann überraschenderweise ohne erhebliche Probleme (Sublimation) durch bloße Fraktionierung geschehen. Die 2,3,4,5-Tetrafluorbenzoesäure geht hierbei bei 99 bis 101 °C (1 mbar) als farblose Flüssigkeit über, die in der Vorlage sofort erstarrt (Ep. 83,1 °C). Auf diese Weise gelangt man problemlos zu den bei den potentiellen Anwendungen der genannten Säure erforderlichen Reinheiten (> 99,9 %).

Die beim erfindungsgemäßen Verfahren als Ausgangsverbindungen der genannten allgemeinen Formel (2) eingesetzen N'-substituierten N-Aminotetrafluorphthalimide können hergestellt werden, indem man 1 Mol 3,4,5,6-Tetrachlorphthalsäureanhydrid mit der mindestens äquimolaren Menge, zweckmäßigerweise mit einem molaren Überschuß bis etwa 20 Mol-%, einer Stickstoffverbindung der allgemeinen Formel (4) 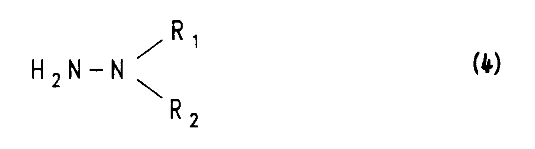
in welcher R₁ und R₂ die vorstehend genannten Bedeutungen haben, in wäßrigalkoholischem Medium, in Eisessig, in etwa 90 bis 100 %iger Schwefelsäure oder in Oleum bei Temperaturen (in Abhängigkeit vom angewandten Medium) von etwa 100 bis 220 °C zum entsprechenden N'-substituierten N-Amino-3,4,5,6-Tetrachlorphthalimid der allgemeinen Formel (3) 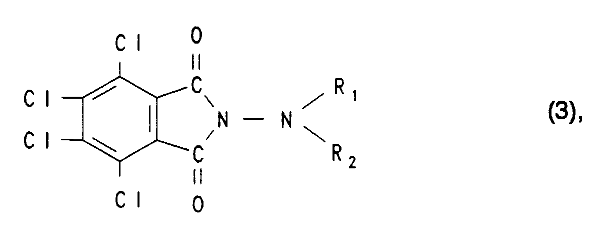
in welcher R₁ und R₂ die vorstehend genannten Bedeutungen haben, umsetzt, das so erhaltene Imid der genannten Formel (3) direkt oder nach erfolgter Umsetzung mit der mindestens äquimolaren Menge Benzaldehyd, welcher am aromatischen Kern beispielsweise durch Fluor- und/oder Chloratome und/oder Alkyl-(C₁-C₄)-Gruppen substituiert sein kann, in an sich bekannter Weise zur entsprechenden Benzalverbindung, oder nach erfolgter Acylierung mit einem Alkyl (C₁-C₆)-CO-halogenid, vorzugsweise -chlorid, Carbonsäureanhydrid der allgemeinen Formel Alkyl(C₁-C₆)-CO-O-OC-(C₁-C₆)Alkyl, einem Aryl-CO-halogenid, vorzugsweise -chlorid, oder Phthalsäureanhydrid, das am aromatischen Ring durch 4 Chloratome oder 4 Fluoratome substituiert sein kann, in an sich bekannter Weise, in einem polar aprotischen Lösungsmittel mit Kalium-, Rubidium- oder Cäsiumfluorid oder Mischungen daraus bei Temperaturen von etwa 50 bis etwa 230 °C, vorzugsweise etwa 90 bis etwa 140 °C, in Gegenwart oder Abwesenheit eines Phasentransferkatalysators umsetzt (Halex-Reaktion).

Geeignete polar aprotische Lösungsmittel für die Fluorierung (Halex-Reaktion) sind beispielsweise Dimethylformamid, Dimethylacetamid, Dimethylsulfoxid, Tetramethylensulfoxid, Dimethylsulfon, Diphenylsulfoxid, Diphenylsulfon, Sulfolan, N-Methylpyrrolidon oder 1,3-Dimethylimidazolidin-2-on.

Als Phasentransferkatalysatoren können beispielsweise quartäre Ammonium- oder Phosphoniumsalze dienen. An geeigneten Verbindungen seien im einzelnen folgende genannt: Tetraalkyl (C₁-C₁₈)-ammoniumchloride oder -bromide, Tetraalkyl-(C₁-C₁₈)-phosphoniumchloride oder -bromide, Tetraphenylphosphoniumchlorid oder -bromid, [(Phenyl)ₘ(alkyl(C₁-C₁₈))ₙ]-phosphoniumchloride oder -bromide, wobei m = 1 bis 3, n = 3 bis 1 und m + n = 4 ist.

Durch die nachstehenden Beispiele wird das erfindungsgemäße Verfahren näher erläutert, ohne darauf beschränkt zu werden.

### Beispiel 1

8,72 g (20 mMol) Oktafluorbisphthalimid wurden in 100 ml Wasser suspendiert und die Lösung mit konzentrierter Schwefelsäure auf pH 0 gestellt. Die Suspension wurde unter Druck 36 h auf 205 °C geheizt. Nach dem Abkühlen wurden eine Suspension erhalten. Durch Filtration erhielt man 3,42 g (17,6 mMol) 2,3,4,5-Tetrafluorbenzoesäure als hellbraunen Feststoff. Durch Extraktion der wäßrigen Phase mit Methyl-tert.butylether, Trocknen über MgSO₄, Filtration und Entfernen des Lösungsmittels erhielt man weitere 3,3 g (17 mMol) an 2,3,4,5-Tetrafluorbenzosäure (Gesamtausbeute, roh, 86 %). Aus mehreren solcher Ansätze wurden die Rohprodukte vereinigt und fraktioniert destilliert, wobei 2,3,4,5-Tetrafluorbenzoesäure bei 99-101 °C (1 mbar) überging.
Schmp. 85,3 °C
¹H-NMR (CDCl₃, interner Standard TMS): δ = 7,69 (dddd, 1H)
¹⁹F-NMR (CDCl₃, interner Standard CFCl₃):
- δ =: -133,2 (dddd, 1F, J = 6,1 Hz, 10,4 Hz, 13,3 Hz, 19,9 Hz)
-137,8 (dddd, 1F, J = 3,3 Hz, 10,5 Hz, 13,1 Hz, 20,9 Hz)
-145,6 (dddd, 1F, J = 8,4 Hz, 10,5 Hz, 19,6 Hz, 20,9 Hz)
-153,3 (dddd, 1F, J = 2,7 Hz, 2,7 Hz, 19,6 Hz, 19,9 Hz)
¹³C-NMR (CDCl₃, interner Standard TMS):
- δ =: 141,6 (ddddd), 144,4 (ddddd), 146,7 (ddddd), 148,8 (ddddd), 167,0 (-COOH) (C-COOH nicht sichtbar)
- MS: m/z (%) =: 45(12), 75(9), 80(14), 99(54), 119(5), 130(12), 149(57), 177(100), 194 (M⁺,82)

### Beispiel 2

6,44 g (20 mMol) N,(N'-Benzyliden)-aminotetrafluorphthalimid wurden in wäßriger Suspension mit 85 prozentiger Phosphorsäure versetzt, bis etwa pH -0,5 erreicht war und anschließend in dieser Mischung 25 h bei 210 °C gehalten. Nach dem Abkühlen wurde die Mischung mit Methyl-tert.butylether extrahiert, die organische Phase mit wäßriger Natronlauge gewaschen und verworfen (Entfernung des Benzaldehydes). Ansäuern der wäßrigen Lösung und erneute Extraktion mittels Methyl-tert.butylether lieferte nach Trocknen und Entfernen des Lösungsmittels 3,23 g (16,6 mMol/83 %) 2,3,4,5-Tetrafluorbenzoesäure, die, wie in Beispiel 1 beschrieben, weiter gereinigt werden kann.

### Beispiel 3

5,24 g (20 mMol) N',N-Dimethylaminotetrafluorphthalimid wurden in 100 ml 5 prozentiger Salzsäure 48 h auf 190 °C erhitzt. Die erhaltene Lösung wurde mit Methyl-tert.-butylether extrahiert und mit gesättigter Kochsalzlösung gewaschen. Trocknen und Entfernen des Lösungsmittels ergab 3,41 g (17,6 mMol/88%) an 2,3,4,5-Tetrafluorbenzoesäure.

Die weitere Reinigung erfolgte wie in Beispiel 1 beschrieben.

### Beispiel 4

Ein Gemisch aus 13,1 (30 mMol) Oktafluorbisphthalimid, 20,4 g Schwefelsäure, 0,75 g Calciumhydroxid in 120 g Wasser (pH 0,5 bei 20° C) wurde 21 h auf 180° C erhitzt. Dabei wurde der Innendruck auf maximal 12 bar begrenzt. Aus der abgekühlten Lösung konnte man durch Extraktion mit Methyl-tert.butylether und anschließender, wie in Beispiel 1 beschriebener Aufarbeitung, 11,2 g (96 %) rohe 2,3,4,5-Tetrafluorbenzoesäure gewinnen, deren Reingehalt (GC, geeicht) 83 % betrug, entsprechend einer Ausbeute von 85 % der Theorie.

Die weitere Reinigung erfolgte wie in Beispiel 1 beschrieben.

## Patentansprüche

1. Verfahren zur Herstellung von 2,3,4,5-Tetrafluorbenzoesäure der Formel (1) 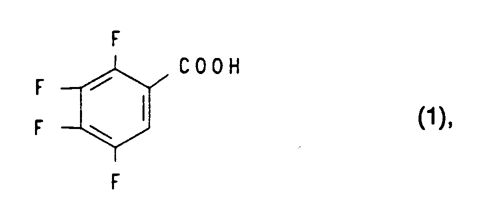 dadurch gekennzeichnet, daß man N'-substituierte N-Aminotetrafluorphthalimide der allgemeinen Formel (2) 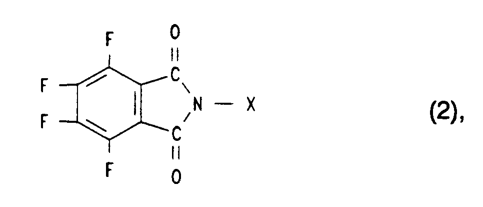 in welcher X den Rest 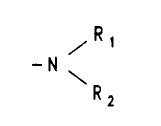 worin R₁, R₂ ein Wasserstoffatom, eine Alkyl (C₁-C₁₀)-Gruppe, Arylgruppe, Alkyl (C₁-C₆)-CO-Gruppe oder Aryl-CO-Gruppe bedeuten, wobei die Aryl- bzw. Aryl-CO-Gruppen für R₁ und R₂ am aromatischen Kern durch Fluor- und/oder Chloratome und/oder Alkyl (C₁-C₄)-Gruppen substituiert sein können, oder R₁ und R₂ zusammen einen Phthaloylrest, der am aromatischen Ring durch 4 Chloratome oder 4 Fluoratome substituiert sein kann, vorzugsweise den Rest 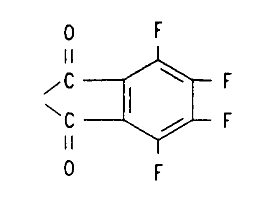 bedeuten,
oder in welcher X den Rest 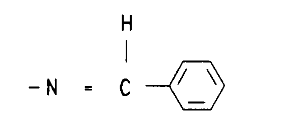 darstellt,
welcher am aromatischen Kern durch Fluor- und/oder Chloratome und/oder Alkyl-(C₁-C₄)-Gruppen substituiert sein kann, in wäßrigem Medium bei pH-Werten von -1 bis +1 bei Temperaturen von 160 bis 220 °C erhitzt.

2. Verfahren nach Anspruch 1, dadurch gekennzeichnet, daß man bei Temperaturen von 190 bis 205 °C erhitzt.

3. Verfahren nach mindestens einem der Ansprüche 1 und 2, dadurch gekennzeichnet, daß man bei pH-Werten von -0,2 bis +0,4 erhitzt.

4. Verfahren nach mindestens einem der Ansprüche 1 bis 3, dadurch gekennzeichnet, daß man den pH-Wert der Reaktionsmischung mit einer organischen Säure, einer Alkyl- oder Arylsulfonsäure oder einer anorganischen Mineralsäure einstellt.

5. Verfahren nach mindestens einem der Ansprüche 1 bis 4, dadurch gekennzeichnet, daß man die angefallene 2,3,4,5-Tetrafluorbenzoesäure durch fraktionierte Destillation reinigt.

6. Verfahren nach mindestens einem der Ansprüche 1 bis 5, dadurch gekennzeichnet, daß man in Gegenwart von Calciumsalzen, Siliciumdioxid oder Trialkyl (C₂-C₆)-zinnchloriden arbeitet.

7. Verfahren nach mindestens einem der Ansprüche 1 bis 6, dadurch gekennzeichnet, daß man in Gegenwart von Calciumhydroxid, Calciumsulfat, Calciumcarbonat, Calciumchlorid oder Siliciumdioxid hoher innerer Oberfläche arbeitet.

## Claims

1. A process for the preparation of 2,3,4,5-tetrafluorobenzoic acid of the formula (1), 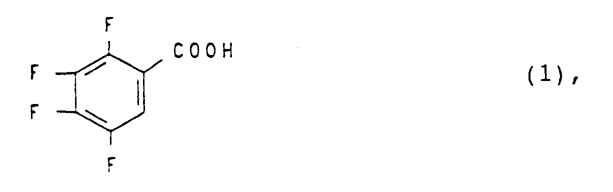 which comprises heating N'-substituted N-aminotetrafluorophthalimides of the formula (2) 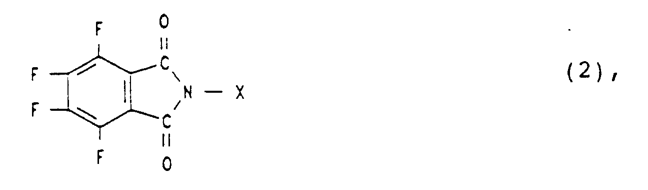 in which X is the radical 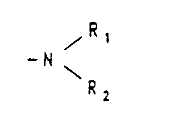 where R₁, R₂ are a hydrogen atom, an alkyl-(C₁-C₁₀) group, aryl group, alkyl-(C₁-C₆)-CO- group or aryl-CO- group, it being possible for the aryl, or aryl-CO-, groups in the case of R₁ and R₂ to be substituted on the aromatic ring by fluorine and/or chlorine atoms and/or alkyl-(C₁-C₄) groups, or R₁ and R₂ together are a phthaloyl radical which can be substituted on the aromatic ring by 4 chlorine atoms or 4 fluorine atoms, preferably the radical 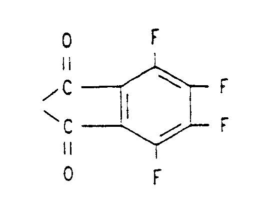 or in which X is the radical 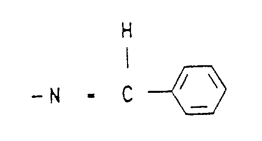 which can be substituted on the aromatic ring by fluorine and/or chlorine atoms and/or alkyl-(C₁-C₄) groups, in an aqueous medium at pH values of -1 to +1 at temperatures of 160 to 220°C.

2. The process as claimed in claim 1, wherein heating is carried out at temperatures of 190 to 205°C.

3. The process as claimed in at least one of claims 1 and 2, wherein heating is carried out at pH values of -0.2 to +0.4.

4. The process as claimed in at least one of claims 1 to 3, wherein the pH of the reaction mixture is adjusted using an organic acid, an alkylsulfonic or arylsulfonic acid or an inorganic mineral acid.

5. The process as claimed in at least one of claims 1 to 4, wherein the 2,3,4,5-tetrafluorobenzoic acid obtained is purified by fractional distillation.

6. The process as claimed in at least one of claims 1 to 5, wherein the reaction is carried out in the presence of calcium salts, silicon dioxide or trialkyl-(C₂-C₆)tin chlorides.

7. The process as claimed in at least one of claims 1 to 6, wherein the reaction is carried out in the presence of calcium hydroxide, calcium sulfate, calcium carbonate, calcium chloride or silicon dioxide with a large internal surface.

## Revendications

1. Procédé de préparation de l'acide 2,3,4,5-tétrafluorobezoïque de formule (1) 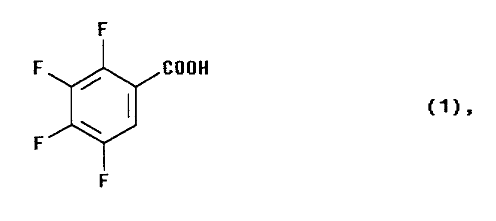 caractérisé en ce que l'on chauffe dans un milieu aqueux, à des températures de 160 à 220°C, à un pH compris entre -1 et +1, des N-aminotétrafluorophtalimides N'-substitués de formule générale (2) 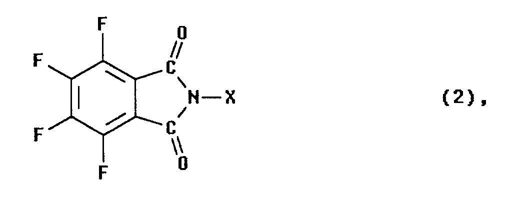 dans laquelle X représente le reste 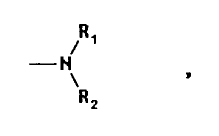 dans lequel R₁, R₂ représentent chacun un atome d'hydrogène, un groupe alkyle en C₁-C₁₀, un groupe aryle, un groupe (alkyle en C₁-C₆)-CO ou un groupe aryl-CO, les groupes aryle ou aryl-CO pour R₁ et R₂ pouvant être substitués sur le noyau aromatique par des atomes de fluor et/ou de chlore et/ou des groupes alkyle en C₁-C₄, ou bien R₁ et R₂ forment ensemble un reste phtaloyle qui peut être substitué sur le noyau aromatique par 4 atomes de chlore ou 4 atomes de fluor, de préférence le reste 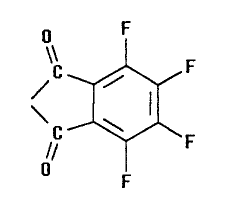 ou bien X représente le reste 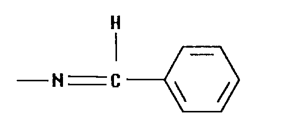 qui peut être substitué sur le noyau aromatique par des atomes de fluor et/ou de chlore et/ou des groupes alkyle en C₁-C₄.

2. Procédé selon la revendication 1, caractérisé en ce que l'on chauffe à des températures de 190 à 205°C.

3. Procédé selon au moins l'une des revendications 1 et 2, caractérisé en ce que l'on chauffe à un pH compris entre -0,2 et +0,4.

4. Procédé selon au moins l'une des revendications 1 à 3, caractérisé en ce que l'on règle le pH du mélange réactionnel avec un acide organique, un acide alkyl-ou arylsulfonique ou un acide minéral inorganique.

5. Procédé selon au moins l'une des revendications 1 à 4, caractérisé en ce que l'on purifie l'acide 2,3,4,5-tétrafluorobezoïque formé par distillation fractionnée.

6. Procédé selon au moins l'une des revendications 1 à 5, caractérisé en ce que l'on travaille en présence de sels de calcium, de dioxyde de silicium ou de chlorures de tri(alkyl en C₂-C₆)- zinc.

7. Procédé selon au moins l'une des revendications 1 à 6, caractérisé en ce que l'on travaille en présence d'hydroxyde de calcium, de sulfate de calcium, de carbonate de calcium, de chlorure de calcium ou de dioxyde de silicium ayant une surface interne élevée.
